(19) Europäisches Patentamt
European Patent Office
Office européen des brevets

(11) **EP 2 203 108 B1**

(12) **EUROPÄISCHE PATENTSCHRIFT**

(45) Veröffentlichungstag und Bekanntmachung des Hinweises auf die Patenterteilung:
**20.04.2011 Patentblatt 2011/16**

(21) Anmeldenummer: **08837256.0**

(22) Anmeldetag: **01.10.2008**

(51) Int Cl.:
***A61B 5/00*** *(2006.01)*

(86) Internationale Anmeldenummer:
**PCT/EP2008/008328**

(87) Internationale Veröffentlichungsnummer:
**WO 2009/046921 (16.04.2009 Gazette 2009/16)**

(54) **VORRICHTUNG ZUM ERKENNEN VON BAKTERIELLEM BEFALL IM WURZELKANAL VON ZÄHNEN**

DEVICE FOR DETECTING SIGNS OF BACTERIAL INFECTION OF THE ROOT CHANNEL OF TEETH

DISPOSITIF POUR DÉTECTER UNE INFECTION BACTÉRIENNE DANS LE CANAL RADICULAIRE DE DENTS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **01.10.2007 DE 102007047068**

(43) Veröffentlichungstag der Anmeldung:
**07.07.2010 Patentblatt 2010/27**

(73) Patentinhaber: **Ferton Holding SA
2800 Delemont (CH)**

(72) Erfinder: **HENNIG, Thomas
40764 Langenfeld (DE)**

(74) Vertreter: **von Kreisler Selting Werner
Deichmannhaus am Dom
Bahnhofsvorplatz 1
50667 Köln (DE)**

(56) Entgegenhaltungen:
JP-A- 3 112 549       US-A- 4 979 900
US-A- 5 503 559       US-A1- 2005 232 550
US-A1- 2007 188 738

• PINI R ET AL: "LASER DENTISTRY ROOT CANAL DIAGNOSTIC TECHNIQUE BASED ON UV-INDUCED FLUORESCENCE SPECTROSCOPY" LASERS IN SURGERY AND MEDICINE, Bd. 9, Nr. 4, 1989, Seiten 358-361, XP002517905 ISSN: 0196-8092

EP 2 203 108 B1

**Beschreibung**

Die Erfindung betrifft eine Vorrichtung zum Erkennen von bakteriellem Befall an Zähnen nach dem Oberbegriff des Anspruchs 1.

Eine derartige Vorrichtung ist aus der DE-A-3031249 bekannt, bei der sichtbare Lumineszenz zur Feststellung des Vorhandenseins von Karies an menschlichen Zähnen verwendet wird. Ein zu untersuchender Zahn wird mit nahezu monochromatischem Licht bestrahlt. An diesem Zahn wird durch das monochromatische Licht eine Fluoreszenzstrahlung angeregt. Die Intensität der Fluoreszenzstrahlung wird bei zwei unterschiedlichen Wellenlängen gemessen und ausgewertet, wobei bei der einen Wellenlänge die Abhängigkeit der Intensität von dem Spektrum für Karies und Nicht-Karies etwa gleich ist, während bei der anderen Wellenlänge die relative Intensität beachtlich beim Vorhandensein von Karies anwächst. Somit kann ein gesunder Zahnbereich von einem kariösen Zahnbereich berührungslos unterschieden werden, indem die Fluoreszenzstrahlung eines mit monochromatischem Licht bestrahlten Zahns erfasst wird.

Aus der EP-A-0862896 ist eine weitere Vorrichtung zum Erkennen von bakteriellem Befall bekannt, bei der mit einem Handstück, das Emissions- und Erfassungseinrichtungen aufweist, um eine Anregungsstrahlung zu erzeugen und diese auf einen zu untersuchenden Zahnbereich zu richten und um die an dem bestrahlten Zahn angeregte Fluoreszenzstrahlung zu erfassen und auszuwerten. Jede Emissions- und Erfassungseinrichtung weist eine Vielzahl von einzelnen Emissionsfasern, welche den zu untersuchenden Zahn mit der Anregungsstrahlung bestrahlen, und Detektionsfasern auf, welche die an dem bestrahlten Zahn angeregte Fluoreszenzstrahlung erfassen.

Eine Vorrichtung zur Untersuchung des Wurzelkanals gemäß dem Oberbegriff des Anspruchs 1 ist ebenfalls aus der US-A-5 503 559 bekannt.

Die bisher bekannten Vorrichtungen haben den Nachteil, dass die Lichtleiter, mit denen die Anregungsstrahlung auf den zu untersuchenden Zahnbereich gerichtet wurde, nicht geeignet sind, in die Wurzelkanäle bis in die Spitzen eingeführt zu werden. Außerdem bestehen die bei den bisher bekannten Vorrichtungen verwendeten Lichtleiter aus Quarzglaslichtleitern, die eine geringe Dehnbarkeit von weniger als 1% aufweisen. Diese Lichtleiter sind somit zu starr, um dem natürlich gebogenen Wurzelkanal zu folgen und brechen in dem rauhen Wurzelkanal sehr schnell ab.

Vor jeder Versiegelung des Wurzelkanals ist es jedoch notwendig, dass der Wurzelkanal vollständig von Bakterien befreit ist.

Der Erfindung liegt demzufolge die Aufgabe zugrunde, eine Vorrichtung der eingangs beschriebenen Art zu schaffen, mit der bakteriell befallene Stellen im Wurzelkanal detektierbar sind.

Zur Lösung dieser Aufgabe dienen die Merkmale des Anspruchs 1.

Die Erfindung sieht in vorteilhafter Weise vor, dass eine Vorrichtung der einleitend beschriebenen Art eine Lichtquelle, eine Empfangseinheit, eine Auswerteeinheit, die mit der Empfangseinheit gekoppelt ist, mindestens eine Emissionsfaser, die mit der Lichtquelle gekoppelt ist, und mindestens eine Detektionsfaser, die mit der Empfangseinheit gekoppelt ist, aufweist. Die gemeinsame distale Stirnfläche der mindestens einen Emissionsfaser und der mindestens einen Detektionsfaser ist mit einer Stirnfläche einer einzelnen flexiblen Kunststofflichtleitfaser verbunden, wobei der Durchmesser der Kunststofflichtleitfaser kleiner als 400 $\mu$m, vorzugsweise kleiner als 300 $\mu$m, sein kann.

Diese Ausführungsform hat den Vorteil, dass die Kunststofflichtleitfaser problemlos bis in die Spitzen des Wurzelkanals eingeführt werden kann, da die Wurzelkanalspitzen in der Regel einen Durchmesser zwischen 300 $\mu$m und 400 $\mu$m aufweisen. Ein weiterer Vorteil ist, dass es sich im Gegensatz zu dem bisher bekannten Stand der Technik um eine einzelne Lichtleitfaser handelt. Die Kunststofflichtleitfaser besteht vorzugsweise aus Polystyrol.

Diese Ausführungsform hat den zusätzlichen Vorteil, dass die flexible Kunststofflichtleitfaser, da sie aus Kunststoff besteht, eine Dehnung von 8% problemlos übersteht und dem Verlauf eines natürlich gebogenen Wurzelkanals problemlos folgen kann. Außerdem ist sie im Gegensatz zu Quarzglaslichtleitern nicht brüchig. Es besteht somit, im Gegensatz zur Verwendung von Quarzglaslichtleitern, nicht die Gefahr, dass Bruchstücke des Lichtleiters im Wurzelkanal bleiben und nur schwer bis gar nicht zu entfernen sind.

Zusätzliche Vorteile sind, dass die Vorrichtung leicht zu fertigen ist, eine einfache Handhabbarkeit sowie eine sichere Anwendung garantiert. Außerdem kann die Kunststofflichtleitfaser leicht ausgewechselt werden.

Bei einer weiteren Ausgestaltung der Erfindung ist vorgesehen, dass die gemeinsame distale Stirnfläche der Emissions- und Detektionsfasern und die proximale Stirnfläche der Kunststofflichtleitfaser mit Hilfe einer Federkraft aneinander gepresst sind.

Als Emissions- und Detektionsfasern können beispielsweise sieben Lichtleitfasern (d.h. drei Emissions- und vier Detektionsfasern oder vier Emissions- und drei Detektionsfaser) mit einem Durchmesser von jeweils 70 $\mu$m oder vierzehn Lichtleitfasern mit einem Durchmesser von jeweils 50 $\mu$m verwendet werden.

Die Kunststofflichtleitfaser kann, insbesondere um den Akzeptanzwinkel zu vergrößern, mehrfach beschichtet sein. Der Kerndurchmesser der Kunststofflichtleitfaser kann kleiner als 350 $\mu$m, vorzugsweise kleiner oder gleich 250 $\mu$m, sein. Der Gesamtdurchmesser der Kunststofflichtleitfaser kann kleiner als 400 $\mu$m, vorzugsweise kleiner als 300 $\mu$m, sein.

Der Kerndurchmesser der Kunststofflichtleitfaser kann gleich oder geringfügig größer sein als der Gesamtdurchmesser der Emissions- und Detektionsfasern.

Als Beschichtungsmaterial können Acrylate bzw. Fluoracrylate mit Brechungsindizes zwischen 1,42 und 1,49 dienen, so dass ein Kunststofflichtleiter mit einem Akzeptanzwinkel von 48° verwirklicht werden kann.

Bei der Erfindung kann die Kunststofflichtleitfaser die von der Lichtquelle über die Emissionsfasern gesendete Anregungsstrahlung zum Zahn und auch die von dem Zahn ausgehende Fluoreszenzstrahlung leiten.

Bei einer weiteren Ausgestaltung der Erfindung ist vorgesehen, dass der Akzeptanzwinkel der Kunststofflichtleitfaser größer als 35° ist.

Alternativ kann der Akzeptanzwinkel der Kunststofflichtleitfaser größer als 40°, vorzugsweise größer als 45°, sein.

Dies hat den Vorteil, dass die Kunststofflichtleitfaser der vorliegenden Erfindung geeignet ist, um auch geradlinige Abschnitte enger Hohlräume zu bestrahlen, ohne dass zusätzliche optische Elemente verwendet werden müssen. Die auf einer ebenen, vorzugsweise senkrecht zur Lichtaustrittsfläche der Kunststofflichtleitfaser verlaufende Fläche erzielte maximale Intensität ist bei einer erfindungsgemäßen Vorrichtung wesentlich höher als bei Vorrichtungen, bei denen übliche Quarzglaslichtleiter verwendet werden.

Der Akzeptanzwinkel der Emissions- und Detektionsfasern kann ebenfalls größer als 35° sein. Alternativ kann der Akzeptanzwinkel auch größer als 40°, vorzugsweise größer als 45°, sein.

Die Emissions- und Detektionsfasern können innerhalb eines Lichtleiterkabels geführt werden. Die Emissions- und Detektionsfasern und somit das Lichtleiterkabel können flexibel sein.

Bei einer Weiterbildung ist vorgesehen, dass die Kunststofflichtleitfaser in einer Inspektionssonde geführt ist, die einen biegsamen bzw. gebogenen Schaft und ein Ankopplungsteil aufweist.

Diese Ausführungsform erleichtert die Handhabbarkeit, da die Kunststofflichtleitfaser aufgrund des biegsamen bzw. gebogenen Schaftes leichter in z. B. Wurzelkanäle eingeführt werden kann.

Der Schaft kann am distalen Ende stumpf ausgeführt sein. Dies reduziert die Verletzungsgefahr.

Die Kunststofflichtleitfaser kann innerhalb des Schafts und/oder Ankopplungsteils verklebt sein.

Die Kunststofflichtleitfaser kann distal 1 bis 30 mm, vorzugsweise 15 bis 25 mm, aus der Inspektionssonde hervorragen.

Der Schaft der Inspektionssonde kann 10 bis 30 mm lang sein, wobei der Durchmesser des Schafts kleiner als 1 mm, vorzugsweise kleiner als 0,7 mm, sein kann. Der Schaft besteht aus Kunststoff oder Metall, vorzugsweise Edelstahl.

Auch kann vorgesehen werden, dass das Ankopplungsteil aus einem standardisierten Spritzenanschluss besteht. In dem Ankopplungsteil kann ein Raum vorgesehen sein, der bei Anschluss der Kunststofflichtleitfaser an die Emissions- und Detektionsfasern einen elastisch gebogenen Teil der Kunststofflichtleitfaser aufnehmen kann.

Die Kunststofflichtleitfaser kann proximal 10 bis 30 mm frei aus der Inspektionssonde hervorragen.

Die Länge der Inspektionssonde kann weniger als 10 cm, vorzugsweise weniger als 7 cm, betragen.

Bei einer Weiterbildung ist vorgesehen, dass eine Zentriervorrichtung mit dem proximalen Ende des Ankopplungsteils verbunden ist, wobei die Kunststofflichtleitfaser in der Zentriervorrichtung geführt ist und zentral am proximalen Ende der Zentriervorrichtung aus dieser hervorragt, wobei die Kunststofflichtleitfaser maximal 2 mm am proximalen Ende der Zentriervorrichtung aus dieser hervorragt.

Eine Weiterbildung ist dadurch gekennzeichnet, dass die Stirnflächen der Kunststofflichtleitfaser entspiegelt sind, wobei die Entspiegelung in Form einer Mottenaugenstruktur verwirklicht ist.

Dieses Ausführungsbeispiel löst das Problem, dass Reflektionen an Lichtaustrittsflächen die Diagnose stören können, da durch diese, neben einer leichten Eigenfluoreszenz der Lichtleiter, ein Hintergrundsignal entsteht.

Bei einer Weiterbildung kann die Kunststofflichtleitfaser als Wechselspitze insbesondere als Einmalartikel ausgebildet sein. Dies hat den Vorteil, dass die Kunststofflichtleitfaser nicht nach jeder Anwendung sterilisiert werden muss.

Bei einer weiteren Ausgestaltung ist vorgesehen, dass die Inspektionssonde an ein Handstück angeschlossen ist und sich die Verbindungsstelle zwischen der gemeinsamen Stirnfläche der Emissions- und Detektionsfasern und der Stirnfläche der Kunststofflichtleitfaser innerhalb des Handstücks befindet.

Dies hat den Vorteil, dass die Vorrichtung besser handhabbar ist, da die Kunststofflichtleitfaser aufgrund des Handstücks besser geführt werden kann.

Bei einer Weiterbildung kann die Lichtquelle innerhalb des Handstücks angeordnet sein, wobei die Gesamtlänge der mindestens einen Emissionsfaser und der Kunststofflichtleitfaser weniger als 60 cm, vorzugsweise weniger als 10 cm, betragen kann.

Dieses Ausführungsbeispiel hat den Vorteil, dass die Anregungsstrahlung keinen langen Weg zurücklegen muss. Aus diesem Grund kann der Intensitätsverlust der Anregungsstrahlung reduziert werden.

Im Folgenden werden unter Bezugnahme auf die Zeichnungen Ausführungsbeispiele der Erfindung näher erläutert.

Es zeigen schematisch:

Fig. 1     eine Seitenansicht auf eine Inspektionssonde, in der ein Bündel aus Emissions- und Detektionsfasern geführt ist,

Fig. 2     eine Seitenansicht auf eine Inspektionssonde, mit gebogenem Schaft und Zentriervorrichtung,

Fig. 3      ein schematisches Blockschaltbild einer erfindungsgemäßen Vorrich- tung,

Fig. 4a     ein Weitwinkellichtleiter, dessen Achse parallel zu einer ebenen Flä- che ausgerichtet ist,

Fig. 4b     ein Quarzglaslichtleiter, der kein Weitwinkellichtleiter ist und dessen Achse parallel zu einer ebenen Fläche ausgerichtet ist,

Fig. 5      eine Darstellung mit Lichtverteilungen auf der ebenen Fläche aus Fig. 4a und Fig. 4b,

Fig. 6      eine Darstellung, die den Zusammenhang zwischen Dämpfung der Beleuchtungsstärke und Länge des Lichtleiters darstellt,

Fig. 7      ein schematisches Blockschaltbild, bei dem die Lichtquelle im Hand- stück angeordnet ist.

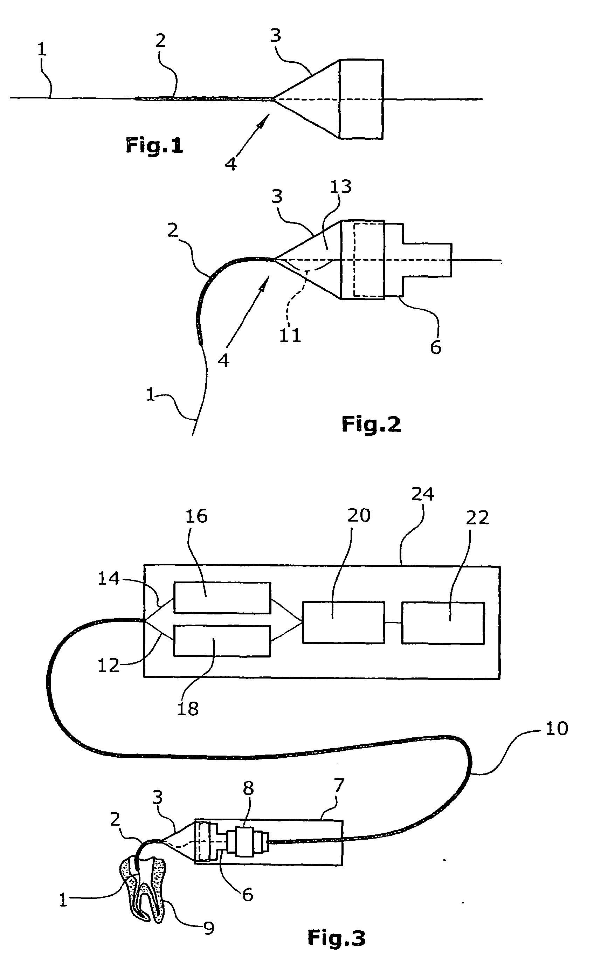

Fig. 1 zeigt eine Inspektionssonde 4. Eine Kunststofflichtleitfaser 1, die insbesondere zum Untersuchen von Wurzelkanälen verwendet wird, ist in einer Inspektionssonde 4 geführt. Die Inspektionssonde 4 besteht aus einem Schaft 2 und einem Ankopplungsteil 3. Distal steht die Kunststofflichtleitfaser 1 gegenüber dem Schaft 2 um 1-30 mm über. Proximal steht die Kunststofflichtleitfaser 1 um 10-30 mm gegenüber dem Ankopplungsteil 3 über. Die Kunststofflichtleitfaser 1 ist vorzugsweise einfach oder mehrfach beschichtet. Der Gesamtdurchmesser der Kunststoffilichtleitfaser 1 liegt unterhalb von 400 $\mu$m, vorzugsweise unterhalb von 300 $\mu$m. Der Kerndurchmesser liegt unterhalb von 300 $\mu$m, vorzugsweise beträgt der Kerndurchmesser 250 $\mu$m. Die Kunststofflichtleitfaser 1 besteht vorzugsweise aus Polystyrol mit einem Brechungsindex von 1,6. Der Schaft 2 besteht vorzugsweise aus Metall oder Kunststoff und ist vorteilhafterweise biegsam ausgeführt. Er kann von dem Benutzer in die gewünschte Form bleibend in der Art einer plastischen Verformung gebogen werden, um einen einfachen Zugang zum Wurzelkanal zu gewährleisten. Die Kunststofflichtleitfaser 1 ist vorzugsweise ein Weitwinkellichtleiter mit einem Akzeptanzwinkel größer als 35°.

Fig. 2 zeigt die Inspektionssonde 4 aus Fig. 1 mit gebogenem Schaft 2. Die Kunststofflichtleitfaser 1 ist im Schaft 2 und/oder am distalen Ende des Ankopplungsteils 3 fixiert bzw. festgeklebt. Am proximalen Ende des Ankopplungsteils 3 ist eine separate Zentriervorrichtung 6 angeschlossen. Die Kunststofflichtleitfaser 1 ist innerhalb der Zentriervorrichtung 6 geführt und ragt am proximalen Ende der Zentriervorrichtung 6 aus dieser hervor. Sie ragt weniger als 2 mm und mehr als 0 mm hervor. Dies ist notwendig, damit ein direkter Kontakt zwischen Kunststofflichtleitfaser 1 und Lichtleiterkabel 10 gewährleistet werden kann, da das proximale Ende der Zentriervorrichtung 6 und damit das proximale Ende der Kunststofflichtleitfaser 1 und das gemeinsame distale Ende der Emissions- und Detektionsfaser mit Hilfe einer Feder gegeneinander gedrückt werden. Wenn nun die Zentriervorrichtung 6 und somit die Kunststofflichtleitfaser 1 mit dem Bündel aus Emissions- 12 und Detektionsfasern 14 in Kontakt ist, wird die Kunststofflichtleitfaser 1 um die proximal der gegenüber der Zentriervorrichtung 6 überstehenden Länge in das Ankopplungsteil 3 zurückgedrückt. Die Kunststofflichtleitfaser 1 kann innerhalb des Ankopplungsteils 3 durch elastische Biegung die Längenänderung wieder ausgleichen, wobei ein gebogener Teil 11 der Kunststofflichtleitfaser 1 in einen Raum 13 ausweichen und eine Rückstellkraft erzeugen kann.

Fig. 3 zeigt ein prinzipielles Blockschaltbild einer erfindungsgemäßen Vorrichtung. Ein Gerät 24 zeigt eine Lichtquelle 18, eine Empfangseinheit 16, eine Auswerteinheit 20 und eine Anzeigeeinheit 22 auf. Die Strahlung aus der Lichtquelle 18 wird vorzugsweise in ihrer Amplitude moduliert, wobei die Modulation mit beispielsweise 2 kHz erfolgt. Die Strahlung aus der Lichtquelle 18 wird in Emissionsfasern 12 eingekoppelt. Die Emissionsfasern 12 werden zusammen mit den Detektionsfasern 14 innerhalb eines Lichtleiterkabels 10 geführt. Die distale Stirnfläche des Bündels aus Emissions- 12 und Detektionsfasern 14 ist mit der proximalen Stirnfläche einer Kunststofflichtleitfaser 1 verbunden. Die Kunststofflichtleitfaser 1 ist innerhalb einer in Fign. 1 und 2 beschriebenen Inspektionssonde 4 geführt. Der Kerndurchmesser der Kunststofflichtleitfaser 1 ist größer oder gleich dem Gesamtdurchmesser der Emissions- 12 und Detektionsfaser 14. Die Kunststofflichtleitfaser 1 ist, wie in Fig. 2 beschrieben, innerhalb einer Zentriervorrichtung 6 geführt und ragt am proximalen Ende der Zentriervorrichtung 6 aus dieser heraus. Die Zentriervorrichtung 6 und somit die Kunststofflichtleitfaser 1 werden mit den Emissions- 12 und Detektionsfasern 14 innerhalb eines Steck- und Kupplungselements 8 durch eine Feder gegeneinander gepresst. Ein solches Steck- und Kupplungselement 8 kann ein handelsüblicher ST-Stecker sein, der eine Bajonetthalterung aufweist. Das Steck- und Kupplungselement 8 befindet sich innerhalb eines Handstücks 7. Die Kunststofflichtleitfaser 1 wird um die gegenüber dem proximalen Ende der Zentriervorrichtung 6 überstehende Länge in das Ankopplungsteil 3 zurückgedrückt. Da die Kunststofflichtleitfaser 1 innerhalb des Schafts 2 und/oder des distalen Endes des Ankopplungsteils 3 fixiert bzw. festgeklebt ist, verbiegt sich die flexible Kunststofflichtleitfaser 1 innerhalb des Raumes 13 des Ankopplungsteils 3. Durch die Biegung steht die Kunststofflichtleitfaser 1 unter Spannung, die bewirkt, dass die Kunststofflichtleitfaser 1 permanent gegen die distale Stirnfläche des Bündels aus Emissions- 12 und Detektionsfasern 14 gedrückt wird. Somit besteht permanent physikalischer Kontakt zwischen der Kunststofflichtleitfaser 1 und dem Bündel aus Emissions- 12 und Detektionsfasern 14. Dies gewährleistet eine gute Einkopplung der Strahlung aus dem Bündel aus Emissions- 12 und Detektionsfasern 14 in die Kunststofflichtleitfaser 1 und umgekehrt. An dieser Ankopplungsstelle wird die Anregungsstrahlung aus den Emissionsfasern 12 in die Kunststofflichtleitfaser 1 eingekoppelt. Das aus der Kunststofflichtleitfaser 1 distal austretende Licht beleuchtet den zu untersuchenden Wurzelkanal, der sich in Zahn 9 befindet. Die Kunststofflichtleitfaser 1 kann bis in die Spitzen des Wurzelkanals eingeführt werden, da der Gesamtdurchmesser der Kunststofflichtleitfaser 1 kleiner als 0,4 mm und die Kunststofflichtleitfaser 1 flexibel ausgeführt ist. Das von dem beleuchteten Wurzelkanalabschnitt zurückgesandte Licht wird vom distalen Ende der Kunststofflichtleitfaser 1 aufgenommen und über die Detektionsfasern 14, die innerhalb des Lichtleiterkabels 10 geführt sind, zu einer Empfangseinheit 16 transportiert. Diese wandelt die empfangene Lichtstrahlung in elektrische Signale um. Die nachgeschaltete Auswerteeinheit 20 vergleicht die Messwerte mit gespeicherten Messwerten von gesunden Zähnen und/oder infizierten Zähnen und es wird ein Verhältnis berechnet. Dieses Verhältnis wird in Form von Werten auf einer Anzeigeeinheit 22 angezeigt. Der Anwender weiß, wenn dieser Wert kleiner als ein bestimmter Wert ist, ist der Wurzelkanalabschnitt frei von bakteriellen Rückständen.

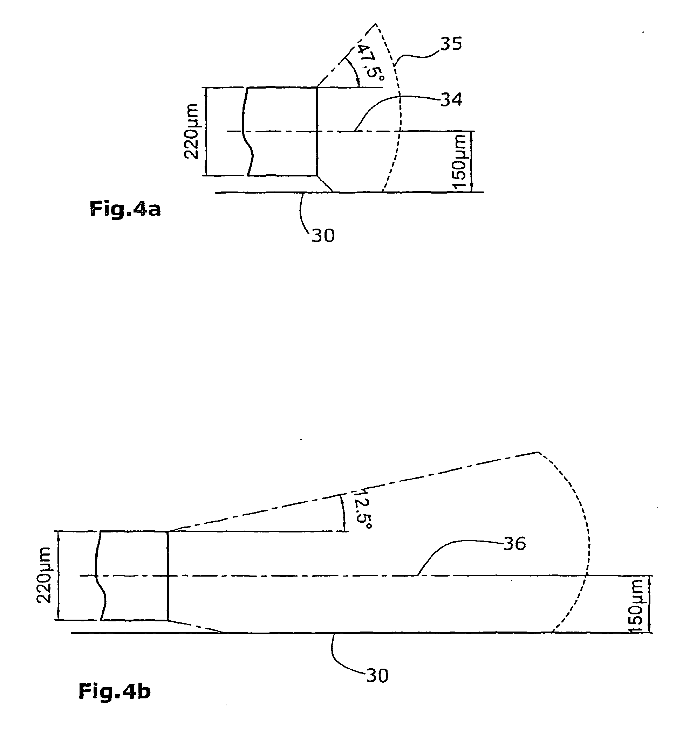

Fign. 4a und 4b zeigen einen Lichtkegel eines Lichtleiters, der einen Weitwinkellichtleiter repräsentiert und im Vergleich hierzu der Lichtkegel eines üblichen Quarzglaslichtleiters, der kein Weitwinkellichtleiter ist. Die Mittelachsen 34,36 der beiden Lichtleiter liegen im Abstand von 150 μm parallel zu einer ebenen Fläche 30. Die Durchmesser der Lichtleiter betragen jeweils 220 μm. Der Weitwinkellichtleiter strahlt das Licht mit einem Öffnungswinkel von 95° ab, was einem Akzeptanzwinkel von 47,5° entspricht. Der herkömmliche Quarzglaslichtleiter, der kein Weitwinkellichtleiter ist, besitzt einen Öffnungswinkel von 25°. Die maximalen auf der ebenen Fläche erzielten Lichtintensitäten liegen in dem Bereichen, in denen die Linien 35 und 37 die ebene Fläche treffen.

In der vorliegenden Erfindung wird vorzugsweise ein Weitwinkellichtleiter mit einem Akzeptanzwinkel größer als 35°, vorzugsweise größer als 40°, verwendet. Es wird vorzugsweise eine Weitwinkel-Kunststofflichtleitfaser aus Polystyrol verwendet.

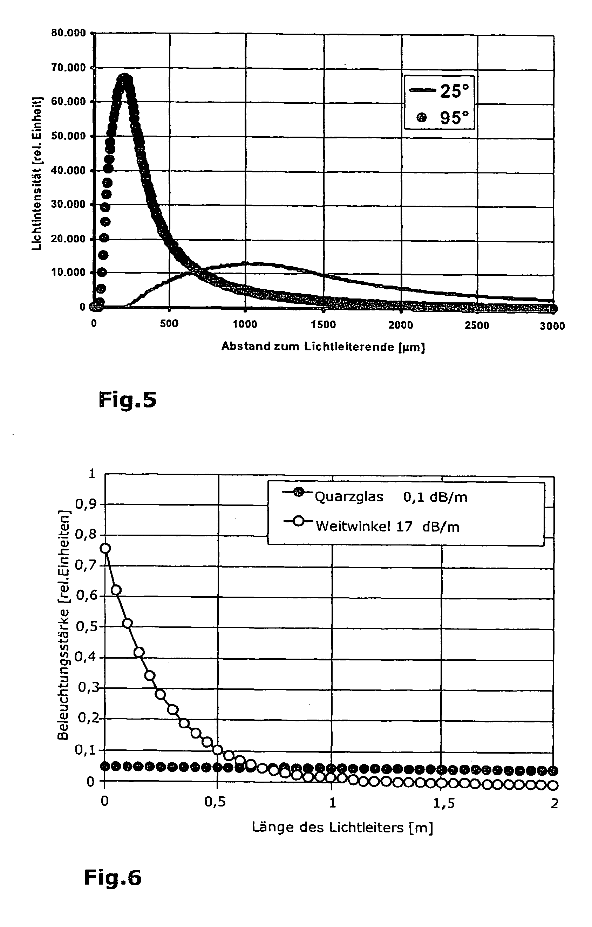

Fig. 5 zeigt die Lichtverteilungen auf der ebenen Fläche 30 aus Fig. 4a und Fig. 4b. Die Lichtaustrittsflächen der Lichtleiter liegen auf der Abszisse beim Wert 0. Die weißen Punkte repräsentieren die Ergebnisse für übliche Quarzglaslichtleiter mit einem Öffnungswinkel von 25°, die schwarzen Quadrate repräsentieren die Ergebnisse für Weitwinkellichtleiter mit einem Öffnungswinkel von 95°. Deutliche Unterschiede zwischen beiden Kurven sind zu erkennen. Ein Öffnungswinkel von nur 25° führt zu einem flachen Kurvenverlauf. Die auf der ebenen Fläche 30 erzielte maximale Lichtintensität liegt etwa 1,1 mm vor dem Lichtleiter mit einem Öffnungswinkel von 25°. Im Falle eines Öffnungswinkels von 95° liegt die auf der ebenen Fläche 30 erzielte maximale Lichtintensität nur etwa 0,2 mm vor dem Lichtleiter. Die auf der ebenen Fläche 30 erzielte maximale Intensität ist im Falle des bei einer erfindungsgemäßen Vorrichtung verwendeten Weitwinkellichtleiters mehr als fünf mal höher im Vergleich zu der maximalen Intensität eines üblichen Quarzglaslichtleiters, der kein Weitwinkellichtleiter ist. Das heißt, es können wesentlich genauere Messwerte ermittelt werden, da das Signal-zu-RauschVerhältnis wesentlich besser ist. Der untersuchte Flächenabschnitt ist bei einem Weitwinkellichtleiter, wie aus Fig. 4a und Fig. 4b ersichtlich, wesentlich kürzer und besser ausgeleuchtet als bei üblichen Quarzglaslichtleitern, die keine Weitwinkellichtleiter sind. Das Verhältnis von bakteriell verunreinigter Fläche zu untersuchtem Flächenabschnitt hat einen direkten Einfluss auf die Messwerte, d.h., wenn die bakteriell verunreinigte Fläche klein ist gegenüber dem untersuchten Flächenabschnitt, so ist die Verunreinigung wegen des geringen prozentualen Anteils der verunreinigten Fläche zu dem insgesamt untersuchten Flächenabschnitt nur schwer aus den Messwerten abzulesen. Kleine Verunreinigungen können somit bei der Verwendung von üblichen Quarzglaslichtleitern mit großem untersuchten Flächenabschnitt und schwacher Ausleuchtung, wie aus Fig. 5 hervorgeht, leicht übersehen werden. Bei Weitwinkellichtleitern mit verhältnismäßig kurzem Flächenabschnitt und intensiver Ausleuchtung ist das Verhältnis von verunreinigter Fläche zu untersuchtem Flächenabschnitt hinsichtlich des prozentualen Anteil günstiger, so dass verunreinigte Flächen eindeutiger und genauer detektierbar ist. Aus diesem Grund können die zu untersuchenden Zahnabschnitte insbesondere in engen Wurzelkanälen mit einem erfindungsgemäßen Weitwinkellichtleiter genauer untersucht werden.

Fig. 6 zeigt die Bestrahlungsstärke am Ende verschiedener Lichtleiterfasern relativ zur Beleuchtungsstärke am Eingang des Lichtleiterkabels 10 in Abhängigkeit von deren Länge. Die relative Beleuchtungsstärke wurde nach der folgenden Formel berechnet:

$$B = NA^2 * 10^{-((a*L)/10)}$$

B: Beleuchtungsstärke
NA: Numerische Apertur
a: Dämpfung des Lichtleiters in dB/m
L: Länge des Lichtleiters in m

Die offenen Kreise symbolisieren einen Weitwinkellichtleiter mit einem Öffnungswinkel von 120°. Dieser Weitwinkellichtleiter weist im Bereich von 400 nm eine Dämpfung von etwa 17 dB/m auf. Die schwarzen Punkte symbolisieren einen Quarzglaslichtleiter mit einem Öffnungswinkel von 25°. Dieser Quarzglaslichtleiter weist im Bereich von 400 nm eine Dämpfung von etwa 0,1 dB/m auf.

Aus Fig. 6 ist ersichtlich, dass lange Lichtleiter, gerade bei Weitwinkellichtleitern, in dem für die Fluoreszenzanregung interessanten, kurzwelligen Spektralbereich um 390 - 420 nm zu einer Abschwächung des an der Austrittsfläche zur Verfügung stehenden Lichts führen. Die Lichtleiter sollten, um diese Dämpfung zu vermeiden, bei Verwendung eines Weitwinkellichtleiters eine Länge von weniger als 60 cm, vorzugsweise weniger als 10 cm, aufweisen. So könnte im Gegensatz zu üblichen Quarzglaslichtleitern, die keine Weitwinkellichtleiter sind, eine etwa zehnfach höhere Beleuchtungsstärke erzielt werden.

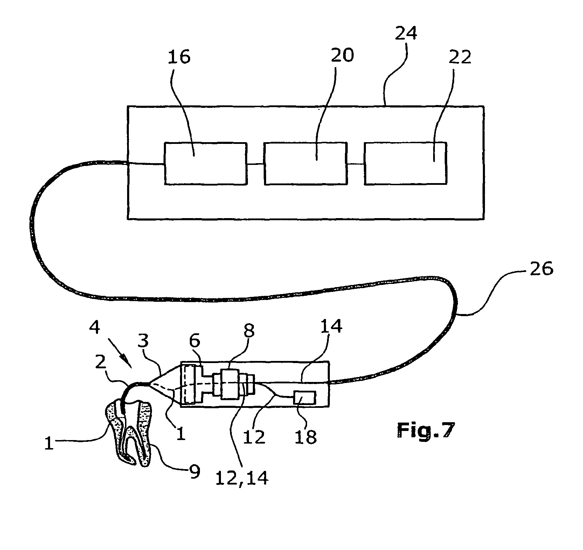

Aus diesem Grund ist in einem bevorzugten Ausführungsbeispiel, das dem Ausführungsbeispiel aus Fig. 3 ähnlich ist, die Lichtquelle 18 innerhalb des Handstücks 7 angeordnet, siehe Fig. 7. Dies hat den Vorteil, dass die Gesamtlänge von Kunststofflichtleitfaser 1 und Emissionsfasern 12 sehr kurz ausführbar ist und damit die Strahlungsverluste der Anregungsstrahlung gering gehalten werden können. Die am Zahn 9 angeregte Fluoreszenzstrahlung wird vom Zahn 9 über die Kunststofflichtleitfaser 1 und die Detektionsfaser 14 zu einer Empfangseinheit 16 gesendet. Die Detektionsfasern 14 werden außerhalb des Handgeräts bis zur Empfangseinheit 16 in einem zweiten Lichtleiterkabel 26 geführt.

## Patentansprüche

1. Vorrichtung zum Erkennen von bakteriellem Befall an Zähnen,

   - mit einer Lichtquelle (18),
   - einer Empfangseinheit (16),
   - einer Auswerteeinheit (20), die mit der Empfangseinheit (16) gekoppelt ist,
   **dadurch gekennzeichnet, dass**
   - mindestens eine Emissionsfaser (12) mit der Lichtquelle (18) gekoppelt ist, und
   - mindestens eine Detektionsfaser (14) mit der Empfangseinheit (16) gekoppelt ist, und

   dass die gemeinsame distale Stirnfläche der mindestens einen Emissionsfaser (12) und der mindestens einen Detektionfaser (14) mit einer Stirnfläche einer einzelnen flexiblen Kunststofflichtleitfaser (1) verbunden ist und dass der Durchmesser der Kunststofflichtleitfaser (1) kleiner als 400 μm, vorzugsweise kleiner als 300 μm ist.

2. Vorrichtung nach Anspruch 1, **dadurch gekennzeichnet, dass** die gemeinsame distale Stirnfläche der Emissions- (12) und Detektionsfasern (14) und die proximale Stirnfläche der Kunststofflichtleitfaser (1) mit Hilfe einer Federkraft aneinandergepresst sind.

3. Vorrichtung nach Anspruch 1 oder 2, **dadurch gekennzeichnet, dass** die Kunststofflichtleitfaser (1) einfach oder mehrfach beschichtet ist.

4. Vorrichtung nach einem der vorhergehenden Ansprüche, **dadurch gekennzeichnet, dass** der Kerndurchmesser der Kunststofflichtleitfaser (1) 300 μm oder kleiner, vorzugsweise 250 μm oder kleiner ist und dass der Gesamtdurchmesser der Kunststofflichtleitfaser kleiner als 400 μm, vorzugsweise kleiner als 300 μm ist.

5. Vorrichtung nach einem der vorhergehenden Ansprüche, **dadurch gekennzeichnet, dass** der Kerndurchmesser der Kunststofflichtleitfaser (1) gleich oder größer ist als der Gesamtdurchmesser der Emissions- (12) und Detektionsfaser (14).

6. Vorrichtung nach einem der vorhergehenden Ansprüche, **dadurch gekennzeichnet, dass** die Kunststofflichtleitfaser (1) aus Polystyrol besteht.

7. Vorrichtung nach einem der vorhergehenden Ansprüche, **dadurch gekennzeichnet, dass** die Kunststofflichtleitfaser (1) die von der Lichtquelle (18) über die Emissionsfasern (12) gesendete Anregungsstrahlung zum Zahn (9) und auch die von dem Zahn (9) ausgehende Fluoreszenzstrahlung leitet.

8. Vorrichtung nach einem der vorhergehenden Ansprüche, **dadurch gekennzeichnet, dass** der Akzeptanzwinkel der Kunststofflichtleitfaser (1) größer als 35°, vorzugsweise größer als 45°, ist.

9. Vorrichtung nach einem der vorhergehenden Ansprüche, **dadurch gekennzeichnet, dass** der Akzeptanzwinkel der Emissions- (12) und Detektionsfasern (14) größer als 35°, vorzugsweise größer als 45°, ist.

10. Vorrichtung nach einem der vorhergehenden Ansprüche, **dadurch gekennzeichnet, dass** die Emissions- (12) und Detektionsfasern (14) und somit das Lichtleiterkabel flexibel sind.

11. Vorrichtung nach einem der vorhergehenden Ansprüche, **dadurch gekennzeichnet, dass** die Kunststofflichtleitfaser (1) in einer Inspektionssonde (4) geführt ist, die einen biegsamen bzw. gebogenen Schaft (2) und einen Ankopplungsteil (3) aufweist.

12. Vorrichtung nach Anspruch 11, **dadurch gekennzeichnet, dass** der Schaft (2) am distalen Ende stumpf ausgeführt ist.

13. Vorrichtung nach Anspruch 11 oder 12, **dadurch gekennzeichnet, dass** die Kunststofflichtleitfaser (1) innerhalb des Schafts (2) und/oder Ankopplungsteils (3) fixiert ist.

14. Vorrichtung nach einem der Ansprüche 11 bis 13, **dadurch gekennzeichnet, dass** die Kunststofflichtleitfaser (1) distal 1-30 mm, vorzugsweise 15-25 mm, aus der Inspektionssonde (4) herausragt.

15. Vorrichtung nach einem der Ansprüche 11 bis 14, **dadurch gekennzeichnet, dass** der Schaft (2) der Inspektionssonde (4) 10-30 mm lang ist.

**Claims**

1. A device for detecting bacterial infection of teeth, comprising

     - a light source (18),
     - a receiving unit (16),
     - an evaluation unit (20) coupled to the receiving unit (16), **characterized in that**
     - at least one emission fiber (12) is coupled to the light source (18), and
     - at least one detection fiber (14) is coupled to the receiving unit (16), and

   that the common distal front face of the at least one emission fiber (12) and of the at least one detection fiber (14) is connected with a front face of a flexible plastic optical waveguide (1) and **in that** the diameter of the plastic optical waveguide (1) is less than 400 μm, preferably less than 300 μm.

2. The device of claim 1, **characterized in that** the common distal front face of the emission fibers (12) and the detection fibers (14) and the proximal front face of the plastic optical waveguide (1) are pressed against each other by spring force.

3. The device of claim 1 or 2, **characterized in that** the plastic optical waveguide (1) has a single coating or multiple coatings.

4. The device of one of the preceding claims, **characterized in that** the core diameter of the plastic optical waveguide (1) is 300 μm or less, preferably 250 μm or less, and that the total diameter of the plastic optical waveguide is less than 400 μm, preferably less than 300 μm.

5. The device of one of the preceding claims, **characterized in that** the core diameter of the plastic optical waveguide (1) is equal to or larger than the total diameter of the emission fiber (12) and the detection fiber (14).

6. The device of one of the preceding claims, **characterized in that** the plastic optical waveguide (1) is made of polystyrene.

7. The device of one of the preceding claims, **characterized in that** the excitation radiation emitted from the light source (18) via the emission fibers (12) is guided to the tooth (9) by said plastic optical waveguide (1) which also guides the fluorescent radiation coming from the tooth (9).

8. The device of one of the preceding claims, **characterized in that** the acceptance angle of the plastic optical waveguide (1) is larger than 35°, preferably larger than 45°.

9. The device of one of the preceding claims, **characterized in that** the acceptance angle of the emission fibers (12) and the detection fibers (14) is larger than 35°, preferably larger than 45°.

10. The device of one of the preceding claims, **characterized in that** the emission fibers (12) and the detection fibers (14), and thus the optical waveguide cable, are flexible.

11. The device of one of the preceding claims, **characterized in that** the plastic optical waveguide (1) is guided in an inspection probe (4) that comprises a flexible or bent shaft (2) and a coupling portion (3).

12. The device of claim 11, **characterized in that** the distal end of the shaft (2) is blunt.

13. The device of claim 11 or 12, **characterized in that** the plastic optical waveguide (1) is fixed within the shaft (2) and/or the coupling portion (3).

14. The device of one of claims 11 to 13, **characterized in that** the plastic optical waveguide (1) projects distally from the inspection probe (4) by 1-30 mm, preferably 15-25 mm.

15. The device of one of claims 11 to 14, **characterized in that** the shaft (2) of the inspection probe (4) is 10-30 mm in length.

**Revendications**

1. Dispositif pour la détection d'une infection bactérienne de dents,

     - comprenant une source de lumière (18),
     - une unité réceptrice (16),
     - une unité d'évaluation (20) couplée à ladite unité réceptrice (16), **caractérisé en ce que**
     - au moins une fibre d'émission (12) est couplée à la source de lumière (18), et
     - au moins une fibre de détection (14) est couplée à ladite fibre d'émission (16), et
     - la face de front commune de ladite au moins une fibre d'émission (12) et de ladite au moins une fibre de détection (14) est connectée à une face de front d'une seule fibre optique (1) flexible en matière plastique, et **en ce que** le diamètre de ladite fibre optique (1) en matière plastique est inférieur à 400 μm, de préférence inférieur à 300 μm.

2. Dispositif selon la revendication 1, **caractérisé en ce que** la face de front commune des fibres d'émission (12) et de détection (14) et la face de front proximale de ladite fibre otique (1) en matière plastique sont pressées l'une contre l'autre par élasticité.

3. Dispositif selon les revendications 1 ou 2, **caractérisé en ce que** ladite fibre optique (1) en matière plastique est recouverte d'une ou plusieurs couches.

4. Dispositif selon l'une quelconque des revendications précédentes, **caractérisé en ce que** le diamètre de l'âme de ladite fibre optique (1) en matière plastique est 300 μm ou inférieur, de préférence 250μm ou inférieur, et **en ce que** le diamètre total de ladite fibre optique en matière plastique est inférieur à 400 μm, de préférence inférieur à 300 μm.

5. Dispositif selon l'une quelconque des revendications précédentes, **caractérisé en ce que** le diamètre de l'âme de ladite fibre optique (1) en matière plastique est égal ou supérieur au diamètre total de ladite fibre d'émission (12) et de détection (14).

6. Dispositif selon l'une quelconque des revendications précédentes, **caractérisé en ce que** ladite fibre optique (1) en matière plastique est fabriquée de polystyrène.

7. Dispositif selon l'une quelconque des revendications précédentes, **caractérisé en ce que** ladite fibre optique (1) en matière plastique guide jusqu'à la dent (9) le rayonnement d'excitation émis par la source de lumière (18) sur les fibres d'émission (12), et aussi guide le rayonnement de fluorescence venant de la dent (9).

8. Dispositif selon l'une quelconque des revendications précédentes, **caractérisé en ce que** l'angle d'acceptance de ladite fibre optique (1) en matière plastique est supérieur à 35°, de préférence supérieur à 45°.

9. Dispositif selon l'une quelconque des revendications précédentes, **caractérisé en ce que** l'angle d'acceptance des fibres d'émission (12) et de détection (14) est supérieur à 35°, de préférence supérieur à 45°.

10. Dispositif selon l'une quelconque des revendications précédentes, **caractérisé en ce que** lesdites fibres d'émission (12) et de détection (14), et pour cela le câble de la fibre optique, sont flexibles.

11. Dispositif selon l'une quelconque des revendications précédentes, **caractérisé en ce que** ladite fibre optique (1) en matière plastique est guidée dans une sonde d'inspection (4) comprenant une tige (2) flexible ou courbée et une partie de raccord (3).

12. Dispositif selon la revendication 11, **caractérisé en ce que** la tige (2) a une extrémité distale émoussée.

13. Dispositif selon les revendications 11 ou 12, **caractérisé en ce que** ladite fibre optique (1) en matière plastique est fixée dans ladite tige (2) et 7 ou ladite partie de raccord (3).

14. Dispositif selon l'une quelconque des revendications 11 à 13, **caractérisé en ce que** ladite fibre optique (1) en matière plastique saille distalement de la sonde d'inspection (4) par 1-30 mm, de préférence 15 - 25 mm.

15. Dispositif selon l'une quelconque des revendications 11 à 14, **caractérisé en ce que** ladite tige (2) de ladite sonde d'inspection (4) a 10-30 mm de longueur.

EP 2 203 108 B1

**Fig.1**

**Fig.2**

**Fig.3**

EP 2 203 108 B1

47,5°

35

34

220µm

150µm

**Fig.4a**

30

12,5°

220µm

36

150µm

**Fig.4b**

30

10

**Fig.5**

**Fig.6**

**Fig.7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3031249 A
- EP 0862896 A
- US 5503559 A